# EUROPEAN PATENT SPECIFICATION

(11) **EP 1 531 789 B1**
(45) Date of publication and mention of the grant of the patent: **17.10.2007**
(21) Application number: 03784614.4
(22) Date of filing: 08.08.2003
(51) Int. Cl.: A61K 8/34, A61K 8/46, A61K 8/89, A61Q 5/02, A61Q 5/12

(54) **HAIR DETERGENT COMPOSITIONS**
SHAMPOOZUSAMMENSETZUNGEN
COMPOSITIONS POUR DETERGENTS CAPILLAIRES

(30) Priority: 09.08.2002 JP 2002232734
(43) Date of publication of application: 25.05.2005
(73) Proprietor: KAO CORPORATION, Tokyo 103-8210 (JP)
(72) Inventor: TERADA, Eiji C/O KAO CORP. RESEARCH LABORATORIES, SUMIDA-KU, Tokyo 131-8501 (JP)
(74) Representative: HOFFMANN EITLE
(86) International application number: PCT/JP2003/010140
(87) International publication number: WO 2004/014334

(56) References cited:
- DE-A- 10 151 592
- US-A- 5 298 240
- US-A- 5 627 148
- US-A1- 2001 056 048
- US-B1- 6 221 817

## Description

### Field of the Invention

The present invention relates to silicone containing hair detergent compositions which provide benefits including rich foaming upon washing the hair and give excellent conditioning effects to the hair.

### Background of the Invention

Although conditioning ingredients such as silicones are usually added to hair detergents for the purpose of giving conditioning effects to the hair, their effects are not always sufficient. Another problem is that they disturb foaming of the detergents.

### Summary of the Invention

In the present invention, there is thus provided a hair detergent composition comprising the following components (a), (b) and (c):
(a) an anionic surfactant,
(b) a monoalkyl glyceryl ether or monoalkenyl glyceryl ether having a C₄₋₁₂ alkyl or alkenyl group, including mixtures thereof, and
(c) a silicone derivative having a group containing both a hydroxy group and a nitrogen atom as a side chain thereof bonded to a silicon atom.

### Detailed Description of the Invention

Hair care compositions are known in the art see for example US 6 221 817, US 5 298 240, US 2001/0056048 and US 5 627 148.

The present invention relates to hair detergent compositions providing rich foaming upon shampooing and is capable of giving excellent conditioning effects to the hair.

The present inventors have found that hair detergent compositions satisfying the above-described demand are obtainable by using, in combination, an anionic surfactant, a specific glyceryl ether, and a silicone derivative having a side chain containing both a hydroxy group and a nitrogen atom.

As the anionic surfactant of Component (a), sulfate-, sulfonate- and carboxylate-type surfactants are preferred. Specific examples include alkyl sulfates, polyoxyalkylene alkyl ether sulfates, polyoxyalkylene alkenyl ether sulfates, alkyl sulfosuccinates, polyoxyalkylene alkyl sulfosuccinates, polyoxyalkylene alkylphenyl ether sulfates, and higher fatty acid salts. Among these, polyoxyalkylene alkyl ether sulfates and alkyl sulfates are preferred, with those represented by the following formula (a1) or (a2) being particularly preferred.

RO(CH₂CH₂O)ₙSO₃M (a1)

R'OSO₃M (a2)

wherein, R represents a C₁₀₋₁₈ alkyl or alkenyl group, R' represents a C₁₀₋₁₈ alkyl group, M represents an alkali metal, alkaline earth metal, ammonium, alkanolamine or basic amino acid, and n stands for a number of from 1 to 5 on weight average.

As Component (a), two or more of these anionic surfactants may be used in combination. The content of Component (a) in the hair detergent composition of the invention preferably ranges from 0.5 to 60 wt.%, more preferably from 1 to 30 wt.%, especially preferably from 8 to 20 wt.%, from the viewpoints of foaming performance, liquid state during use and detergency.

The alkyl or alkenyl group of the monoalkyl glyceryl ether or monoalkenyl glyceryl ether as Component (b) is preferably a linear or branched C₄₋₁₀, especially C₈₋₁₀, alkyl group. Specific examples include n-butyl, isobutyl, n-pentyl, 2-methylbutyl, isopentyl, n-hexyl, isohexyl, n-heptyl, n-octyl, 2-ethylhexyl, n-decyl and isodecyl groups, of which 2-ethylhexyl and isodecyl groups are especially preferred.

As Component (b), two or more of the above-described compounds may be used in combination. The content of Component (b) in the hair detergent composition of the invention ranges preferably from 0.1 to 30 wt.%, more preferably from 0.5 to 15 wt.%, especially preferably from 1 to 10 wt.%, from the viewpoints of attaining sufficient foaming power without impairing a feeling upon use and conditioning effects.

The silicone derivative as Component (c) has, as a side chain thereof bonded to a silicon atom, a group containing both a hydroxy group and a nitrogen atom. Preferred specific examples include those represented by the following average formula (1): 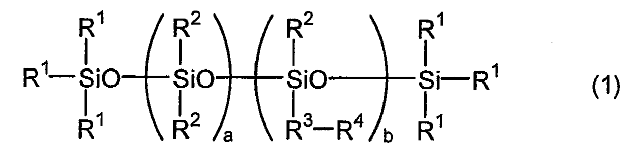 wherein, R¹ each independently represents a monovalent hydrocarbon group, a hydroxy group or an alkoxy group,
R² each independently represents a monovalent hydrocarbon group,
R³ each independently represents a divalent C₁₋₁₀ hydrocarbon group,
R⁴ each independently represents a group represented by the following formula (2) or (3): 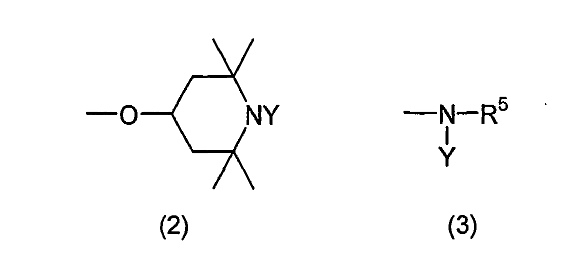 wherein, Y each independently represents a hydrogen atom or a group: -CH₂CH(OH)-R³-OH (R³ has the same meaning as described above), R⁵ each independently represents a hydrogen atom or a group -R³NY₂ (Y and R³ have the same meanings as described above), with the proviso that all the Ys do not represent a hydrogen atom simultaneously,
a stands for a number of from 25 to 1,000, and
b stands for a number of from 1 to 200.

Examples of the monovalent hydrocarbon group as R¹ include alkyl groups and aryl groups. As R¹, C₁₋₃ alkyl groups (particularly, methyl group) and C₁₋₁₅, especially C₁₀₋₁₅ alkoxy groups are preferred.

Examples of the monovalent hydrocarbon group as R² include C₁₋₆ alkyl groups such as methyl, ethyl, propyl, butyl, pentyl and hexyl, C₆₋₁₀ aryl groups such as phenyl, tolyl and xylyl, and C₆₋₁₀ aralkyl groups such as benzyl and phenethyl. Among these, an alkyl group, particularly a methyl group, is preferred.

Examples of the divalent C₁₋₁₀ hydrocarbon group as R³ include methylene group, alkylene groups such as ethylene, trimethylene, propylene, tetramethylene, methyltrimethylene, ethylethylene and dimethylethylene groups, and alkylenearylene groups such as a group represented by the formula: -(CH₂)₂-C₆H₄-. Among these, C₂₋₄ alkylene groups are preferred.

When Y represents a group: -CH₂CH(OH)-R³-OH, it is preferably a 2,3-dihydroxypropyl group. As R⁴, groups represented by the formula (3) are preferred, while as R⁵, N-(2,3-dihydroxypropyl)aminoethyl and N,N-bis(2,3-dihydroxypropyl)aminoethyl groups are preferred.

It is preferred that a stands for a number of from 75 to 400 and b stands for a number of from 1 to 20.

The silicone derivative serving as Component (c) can be synthesized, for example, by reacting an amino-modified silicone with an epoxy functional compound such as glycidol as described in EP-0399706A2. Examples of the silicone derivative as Component (c) include compounds represented by the below-described formula, while those of commercially available products include "8500 Conditioning Agent" (CAS No. 237753-63-8; product of Dow Corning Corp). 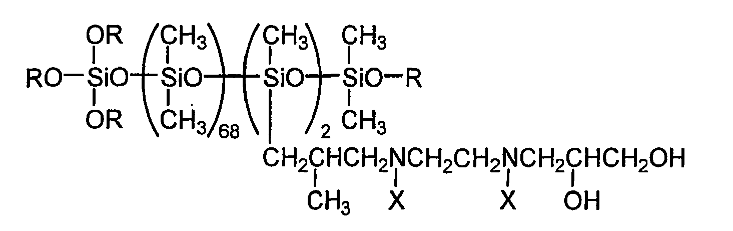 R: C₁₃H₂₇ to C₁₅H₃₁
X: 75% of -CH₂CH(OH)CH₂OH and 25% of hydrogen atom

As Component (c), two or more of the above-described derivatives may be used in combination. From the viewpoints of smoothness and softness of the hair during the period of time from shampooing to rinsing, and smoothness of the hair after drying, the content of Component (c) in the detergent composition of the invention ranges preferably from 0.05 to 4 wt.%, more preferably from 0.07 to 2 wt.%, especially preferably from 0.1 to 1.5 wt.%.

To the detergent composition of the invention, a nonionic surfactant or amphoteric surfactant may be added in order to improve foaming performance.

Examples of the nonionic surfactant include polyoxyalkylene sorbitan fatty acid esters, polyoxyalkylene sorbit fatty acid esters, polyoxyalkylene glycerin fatty acid esters, polyoxyalkylene fatty acid esters, polyoxyalkylene alkyl ethers, polyoxyalkylene alkyl phenyl ethers, polyoxyalkylene (hydrogenated) castor oils, sucrose fatty acid esters, polyglycerin alkyl ethers, polyglycerin fatty acid esters, fatty acid alkanolamides and alkyl glycosides. Among these, alkyl glycosides, polyoxyalkylene alkyl ethers, polyoxyalkylene (C₈ to C₂₀) fatty acid esters, polyoxyethylene sorbitan fatty acid esters, polyoxyethylene hydrogenated castor oils, and fatty acid alkanolamides are preferred. As fatty acid alkanolamides, those having a C₈₋-₁₈, especially C₁₀₋₁₆, acyl group are preferred. The fatty acid alkanolamides may be either monoalkanolamides or dialkanolamides, and those having a C₂₋₃ hydroxyalkyl group are preferred. Examples include oleic diethanolamide, palm kernel fatty acid diethanolamide, coconut fatty acid diethanolamide, lauric diethanolamide, polyoxyethylene coconut fatty acid monoethanolamide, coconut fatty acid monoethanolamide, lauric isopropanolamide and lauric monoethanolamide.

As the amphoteric surfactant, betaine surfactants are usable. Among these, alkyldimethylaminoacetic betaines and fatty acid amidopropyl betaines are more preferred, with fatty acid amidopropyl betaines being particularly preferred. The fatty acid amidopropyl betaines preferably have a C₈₋₁₈, especially C₁₀₋₁₆, acyl group. Among these, lauramidopropyl betaine, palm kernel amidopropyl betaine and cocamidopropyl betaine are especially preferred.

To the detergent composition of the present invention, a conditioning component selected from cationic polymers, cationic surfactants, silicones other than Component (c) and oils can be added in order to improve the finish after drying.

Examples of the cationic polymer include cationized cellulose derivatives, cationic starch, cationized guar gum derivatives, homopolymers of diallyl quaternary ammonium salts, diallyl quaternary ammonium salt/acrylamide copolymers, quaternized polyvinylpyrrolidone derivatives, polyglycol polyamine condensates, vinylimidazolium trichloride/vinylpyrrolidone copolymers, hydroxyethyl cellulose/dimethyldiallylammonium chloride copolymers, vinylpyrrolidone/quaternized dimethylaminoethyl methacrylate copolymers, polyvinylpyrrolidone/alkylamino acrylate copolymers, polyvinylpyrrolidone/alkylamino acrylate/vinylcaprolactam copolymers, vinylpyrrolidone/methacrylamidopropyl trimethylammonium chloride copolymers, alkylacrylamide/acrylate/alkylaminoalkylacrylamide/polyethy lene glycol methacrylate copolymers, an adipic acid/dimethylaminohydroxypropyl ethylenetriamine copolymer ("Cartaretin", product of Sandoz/USA), and cationic polymers as described in Japanese Patent Laid-Open Nos. 139734/1978 and 36407/1985. Among these, cationized cellulose derivatives and cationized guar gum derivatives are particularly preferred.

Examples of the cationic surfactant include lauryl trimethyl ammonium chloride, cetyl trimethyl ammonium chloride, cetyl trimethyl ammonium bromide, stearyl trimethyl ammonium chloride, stearyl trimethyl ammonium bromide, lauryl trimethyl ammonium bromide, behenyl trimethyl ammonium chloride, dialkyl dimethyl ammonium chloride, dicetyl dimethyl ammonium chloride, distearyl dimethyl ammonium chloride, dicocoyl dimethyl ammonium chloride, myristyl dimethyl benzyl ammonium chloride, stearyl dimethyl benzyl ammonium chloride, lanolin fatty acid amidopropyl ethyldimethyl ammonium ethyl sulfate, lanolin fatty acid amidoethyl triethyl ammonium ethyl sulfate, stearyl amidopropyl dimethylamine (and salts thereof), stearyl amidoethyl diethylamine (and salts thereof), stearoxy propyl dimethylamine (and salts thereof), stearoxy propyl trimethyl ammonium chloride, lanolin fatty acid amidopropyl triethyl ammonium ethyl sulfate, lanolin fatty acid amidoethyl trimethyl ammonium methyl sulfate, lanolin fatty acid amidopropyl ethyldimethyl ammonium methyl sulfate, isoalkanoic acid (C₁₄₋₂₀) amidopropyl ethyldimethyl ammonium ethyl sulfate, isoalkanoic acid (C₁₈-22) amidopropyl ethyldimethyl ammonium ethyl sulfate, isostearic acid amidopropyl ethyldimethyl ammonium ethyl sulfate, isononanoic acid amidopropyl ethyldimethyl ammonium ethyl sulfate and alkyl trimethyl ammonium saccharine.

As the silicones other than Component (c), the following compounds can be given as examples.

### (Silicones-1) Dimethylpolysiloxane

Examples include compounds represented by the following formula:

(Me)₃SiO-[(Me)₂SiO]_{d}-Si(Me)₃

wherein, Me represents a methyl group and d stands for a number of from 3 to 2,000.

### (Sililcones-2) Amino-modified silicone

Various amino-modified silicones are usable, but those having an average molecular weight of from about 3,000 to 100,000 and described under the name of Amodimethicone in the CTFA Dictionary (Cosmetic Ingredient Dictionary, USA), third edition are particularly preferred. This amino-modified silicone is preferably employed in the form of an aqueous emulsion and "SM 8704C" (product of Dow Corning Toray Silicone), "DC 929" (product of Dow Corning), etc. are the commercially available products of the aqueous emulsion.

### (Silicones-3) The other silicones

In addition to the above-described silicones, usable are polyether-modified silicones, methylphenyl polysiloxane, fatty acid-modified silicones, alcohol-modified silicones, alkoxy-modified silicones, epoxy-modified silicones, fluorine-modified silicones, cyclic silicones, and alkyl-modified silicones.

The term "oils" to be used herein as the conditioning component means an oily substance other than silicones and examples include hydrocarbons such as squalene, squalane, liquid paraffin, liquid isoparaffin and cycloparaffin; glycerides such as castor oil, cacao oil, mink oil, avocado oil and olive oil; waxes such as beeswax, spermaceti, lanolin and carnauba wax; alcohols such as cetyl alcohol, oleyl alcohol, stearyl alcohol, isostearyl alcohol, 2-octyldodecanol and glycerin; esters such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, coconut fatty acid, isostearic acid and isopalmitic acid; and isostearyl glyceryl ether and polyoxypropylene butyl ether. Among these, esters, particularly hexadecyl 2-ethylhexanoate, isononyl isononanoate and isopropyl palmitate, are particularly preferred.

As the conditioning component, two or more of these compounds may be used in combination. Its content in the hair detergent composition of the invention ranges preferably from 0.05 to 10 wt.%, more preferably from 0.07 to 5 wt.%, especially preferably from 0.1 to 2 wt.% from the viewpoints of lubrication of foams, and smoothness during the period of time from shampooing to rinsing.

In addition to the above-described components, components conventionally used for a hair detergent can be incorporated in the hair detergent composition of the present invention, depending on the purpose. Such optional components include antidandruff, vitamins, bactericides, anti-inflammatory agents, antiseptics, chelating agents, humectants such as sorbitol and pantenol, colorants such as dyes and pigments, viscosity regulators such as hydroxyethyl cellulose, methyl cellulose, polyethylene glycol and clay minerals; pH regulators such as citric acid and potassium hydroxide; vegetable extracts; pearling agents; perfumes; coloring matters; ultraviolet absorbers; antioxidants; and the other components as described in the ENCYCLOPEDIA OF SHAMPOO INGREDIENTS (MICELLE PRESS).

Although the form of the hair detergent of the invention can be selected as needed from liquid, powder, gel and granule, a liquid type using water or a lower alcohol, especially water, as a solvent is preferred.

The hair detergent of the invention is preferably used as a shampoo composition.

The hair detergent of the invention preferably has a pH of from 3 to 10, especially preferably from 3 to 7 when diluted to 20 times the weight with water.

### -Examples-

The following examples further describe and demonstrate embodiments of the present invention. The examples are given solely for the purpose of illustration and are not to be construed as limitations of the present invention.

### Example 1 and Comparative Examples 1 to 3

Shampoo compositions as shown in Table 1 were prepared and they were organoleptically evaluated.

### (Hair washing method)

After the hair was moistened sufficiently, 5 g or 10 g (5 g for medium-length hair and 10 g for long hair) of the shampoo composition was applied to the hair and the hair was washed therewith. The hair was then rinsed well with water, followed by sufficient drying with hot air from a dryer.

### (Organoleptic Evaluation)

The shampoo compositions were evaluated by a panel of 10 experts based on the criteria described below and ranked based on the average score.

### Evaluation criteria

(1) Foaming performance
   4: Excellent foaming
   3: Good foaming
   2: Average foaming
   1: Little foaming
   0: No foaming
(2) Softness of the hair during foaming
   4: Very soft
   3: Soft
   2: Slightly soft
   1: Not so soft
   0: Rigid
(3) Smoothness of the hair during rinsing and after drying
   4: Very smooth
   3: Smooth
   2: Slightly smooth
   1: Not so smooth
   0: Not smooth

### Rank

A: an average score of not less than 3.5
B: an average score of not less than 2.5 but less than 3.5
C: an average score of not less than 1.5 but less than 2.5
D: an average score less than 1.5

**Table 1**

| Composition (wt.%) | | Examples | Comparative Examples | | |
|---|---|---|---|---|---|
| | | 1 | 1 | 2 | 3 |
| (a) | Sodium polyoxyethylene (2) lauryl ether sulfate | 10.0 | 10.0 | 10.0 | 10.0 |
| (b) | 2-Ethylhexyl glyceryl ether | 2.0 | 2.0 | - | 2.0 |
| (c) | Silicone derivative * | 0.5 | - | 0.5 | - |
| Others | Amino-modified silicone | - | - | - | 0.5 |
| | ("KT1989", product of GE Toshiba Silicones) | | | | |
| | Cocamidopropyl betaine | 3.0 | 3.0 | 3.0 | 3.0 |
| | Cocamide MEA | - | - | 2.0 | - |
| | Ethylene glycol distearyl ester | 1.0 | 1.0 | 1.0 | 1.0 |
| | Cationized cellulose ("UCare Polymer JR-400", product of Amerchol) | 0.5 | 0.5 | 0.5 | 0.5 |
| | Sodium chloride | 0.5 | 0.5 | 0.5 | 0.5 |
| | Perfume | Trace | Trace | Trace | Trace |
| | Citric acid | q.s. | q.s. | q.s. | q.s. |
| | Purified water | Balance | Balance | Balance | Balance |
| pH (after diluted to 20 times the weight) | | 6.0 | 6.0 | 6.0 | 6.0 |
| Evaluation | Foaming performance | A | A | C | B |
| | Softness of hair during foaming | A | C | B | B |
| | Smoothness of hair during rinsing | A | C | B | D |
| | Smoothness of hair after drying | A | C | B | C |

| | | | | | |
|---|---|---|---|---|---|
| *silicone derivative: 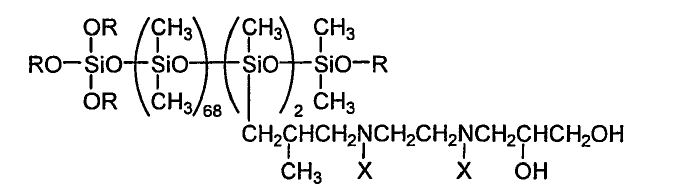 R: C₁₃H₂₇ to C₁₅H₃₁ X: 75% of -CH₂CH(OH)CH₂OH and 25% of hydrogen atom | | | | | |

### Example 2: Conditioning shampoo

| | (wt.%) |
|---|---|
| Sodium polyoxyethylene (2) lauryl ether sulfate | 11.0 |
| Isodecyl glyceryl ether | 1.5 |
| Silicone derivative ^{*1} | 1.0 |
| Cocamidopropyl betaine | 3.0 |
| Ethylene glycol distearyl ester | 1.0 |
| Cationized guar gum | |
| ("Jaguar C-13S", product of RHODIA) | 0.4 |
| Sodium chloride | 1.0 |
| Polypropylene glycol (Mw=400) | 1.0 |
| Malic acid | 1.0 |
| Perfume | trace |
| Aqueous solution of sodium hydroxide | q.s. |
| Purified water | Balance |

| | |
|---|---|
| *1: sold from Dow Corning under the name of "8500 CONDITIONING AGENT". It contains, as an effective ingredient, 60 wt.% of a silicone derivative (CAS No. 237753-63-8) having a group containing both a hydroxy group and a nitrogen atom as a side chain. | |

It has been found that the shampoo thus obtained (having pH 3.7 when diluted to 20 times the weight) had excellent foaming performance and at the same time had high hair conditioning effects.

### Example 3: Conditioning shampoo

| | (wt. %) |
|---|---|
| Sodium polyoxyethylene (2) lauryl ether sulfate | 8.0 |
| Sodium lauryl sulfate | 5.0 |
| Isodecyl glyceryl ether | 1.5 |
| Silicone derivative ^{*1} | 0.5 |
| Dimethyl polysiloxane (viscosity: 100,000 mPa·s) | 0.3 |
| Cocamide MEA | 1.0 |
| Myristyl alcohol | 1.0 |
| Cetanol | 0.5 |
| Glycerin | 1.0 |
| Ethylene glycol distearyl ester | 1.0 |
| Cationized cellulose ("UCare Polymer JR-30M", product of Amerchol) | 0.5 |
| Sodium chloride | 0.5 |
| Benzyloxy ethanol | 1.0 |
| Lactic acid | 1.0 |
| Perfume | trace |
| Aqueous solution of sodium hydroxide | q.s. |
| Purified water | Balance |

| | |
|---|---|
| *1: sold from Dow Corning under the name of "8500 CONDITIONING AGENT". It contains, as an effective ingredient, 60 wt.% of a silicone derivative (CAS No. 237753-63-8) having a group containing both a hydroxy group and a nitrogen atom as a side chain. | |

It has been found that the shampoo thus obtained (having a pH of 3.9 when diluted to 20 times the weight) had an excellent foaming performance, and at the same time had high hair conditioning effects.

## Claims

1. A hair detergent composition comprising the following components (a), (b) and (c):
(a) an anionic surfactant,
(b) a monoalkyl glyceryl ether having a C₄₋₁₂ alkyl group, a monoalkenyl glyceryl ether having a C₄₋₁₂ alkenyl group, or mixtures thereof and
(c) a silicone derivative having a group containing both a hydroxy group and a nitrogen atom as a side chain thereof bonded to a silicon atom.

2. The hair detergent composition of Claim 1,
wherein the anionic surfactant as Component (a) is selected from the group consisting of sulfate-, sulfonate-, carboxylate-type, and mixtures thereof.

3. The hair detergent composition of Claim 1,
wherein the anionic surfactant as Component (a) is selected from the group consisting of RO(CH₂CH₂O)ₙSO₃M, R'OSO₃M, and mixtures thereof wherein, R represents a C₁₀₋₁₈ alkyl or alkenyl group, R' represents a C₁₀₋₁₈ alkyl group, M represents an alkali metal, alkaline earth metal, ammonium, alkanolamine or basic amino acid, and n stands for a number of from 1 to 5 on weight average.

4. The hair detergent composition of Claim 1,
wherein component (b) is a mono alkyl glyceryl ether having a linear C₄₋₁₀ alkyl group, a mono alkyl glyceryl ether having a branched C₄₋₁₀ alkyl group, or mixtures thereof.

5. The hair detergent composition of Claim 4,
wherein alkyl group is selected from the group consisting of n-butyl, isobutyl, n-pentyl, 2-methylbutyl, isopentyl, n-hexyl, isohexyl, n-heptyl, n-octyl, 2-ethylhexyl, n-decyl and isodecyl groups.

6. The hair detergent composition of Claim 1,
wherein the silicone derivative as component (c) is represented by the average formula (1) below 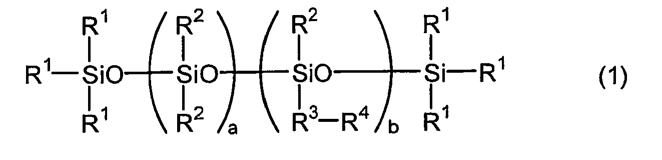 wherein, R¹ each independently represents a monovalent hydrocarbon group, a hydroxy group or an alkoxy group,
R² each independently represents a monovalent hydrocarbon group,
R³ each independently represents a divalent C₁₋₁₀ hydrocarbon group,
R⁴ each independently represents a group represented by the following formula (2) or (3): 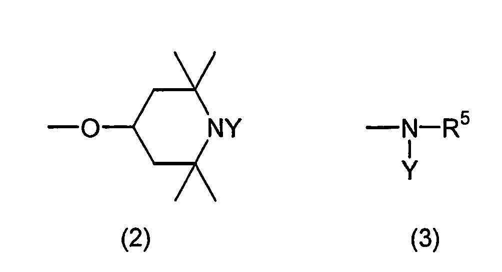 wherein, Y each independently represents a hydrogen atom or a group: -CH₂CH(OH)-R³-OH (R³ has the same meaning as described above), R⁵ each independently represents a hydrogen atom or a group -R³NY₂ (Y and R³ have the same meanings as described above), wherein all the Ys do not represent a hydrogen atom simultaneously,
a stands for a number of from 25 to 1,000, and
b stands for a number of from 1 to 200.

7. The hair detergent composition comprising the following components (a), (b), and (c):
(a) from 0.5% to 60 wt.% of an anionic surfactant,
(b) from 0.1% to 30 wt.% of a monoalkyl glyceryl ether or monoalkenyl glyceral ether having a C₄₋₁₂ alkyl or alkenyl group, including mixtures thereof, and
(c) from 0.05% to 4 wt.% of a silicone derivative having a group containing both a hydroxy group and a nitrogen atom as a side chain thereof bonded to a silicon atom.

8. The hair detergent composition of Claim 7,
wherein the silicone derivative as Component (c) is represented by the average formula (1) below 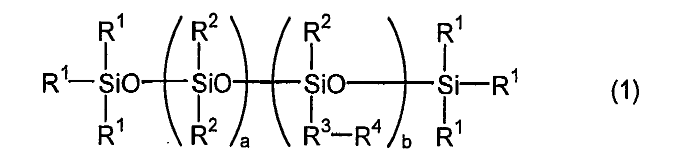 wherein, R¹ each independently represents a monovalent hydrocarbon group, a hydroxy group or an alkoxy group,
R² each independently represents a monovalent hydrocarbon group,
R³ each independently represents a divalent C₁₋₁₀ hydrocarbon group,
R⁴ each independently represents a group represented by the following formula (2) or (3): 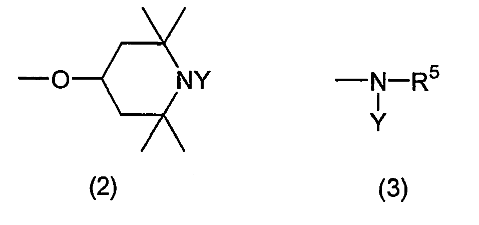 wherein, Y each independently represents a hydrogen atom or a group: -CH₂CH(OH)-R³-OH (R³ has the same meaning as described above), R⁵ each independently represents a hydrogen atom or a group -R³NY₂ (Y and R³ have the same meanings as described above), wherein all the Ys do not represent a hydrogen atom simultaneously,
a stands for a number of from 25 to 1,000, and
b stands for a number of from 1 to 200.

## Patentansprüche

1. Haarreinigungszusammensetzung, umfassend die folgenden Komponenten (a), (b) und (c):
(a) ein anionisches Tensid,
(b) einen Monoalkylglycerylether mit einer C₄₋₁₂-Alkylgruppe, einen Monoalkenylglycerylether mit einer C₄₋₁₂-Alkenylgruppe oder Mischungen davon, und
(c) ein Siliconderivat mit einer Gruppe, die sowohl eine Hydroxygruppe als auch ein Stickstoffatom als Seitenkette davon enthält, die an ein Siliciumatom gebunden ist.

2. Haarreinigungszusammensetzung nach Anspruch 1, worin das anionische Tensid als Komponente (a) ausgewählt ist aus der Gruppe bestehend aus dem Sulfat-, Sulfonat-, Carboxylat-Typ und Mischungen davon.

3. Haarreinigungszusammensetzung nach Anspruch 1, worin das anionische Tensid als Komponente (a) ausgewählt ist aus der Gruppe bestehend aus RO(CH₂CH₂O)ₙSO₃M, R'OSO₃M und Mischungen davon, worin R eine C₁₀₋₁₈-Alkyl- oder -Alkenylgruppe ist, R' eine C₁₀₋₁₈-Alkylgruppe ist, M ein Alkalimetall, Erdalkalimetall, Ammonium, Alkanolamin oder eine basische Aminosäure ist und n für eine Zahl von im Gewichtsmittel 1 bis 5 steht.

4. Haarreinigungszusammensetzung nach Anspruch 1, worin die Komponente (b) ein Monoalkylglycerylether mit einer linearen C₄₋₁₀-Alkylgruppe, ein Monoalkylglycerylether mit einer verzweigten C₄₋₁₀-Alkylgruppe oder eine Mischung davon ist.

5. Haarreinigungszusammensetzung nach Anspruch 4, worin die Alkylgruppe ausgewählt ist aus der Gruppe bestehend aus n-Butyl-, Isobutyl-, n-Pentyl-, 2-Methylbutyl-, Isopentyl-, n-Hexyl-, Isohexyl-, n-Heptyl-, n-Octyl-, 2-Ethylhexyl-, n-Decyl- oder Isodecylgruppen.

6. Haarreinigungszusammensetzung nach Anspruch 1, worin das Siliconderivat als Komponente (c) durch die durchschnittliche Formel (1) dargestellt ist 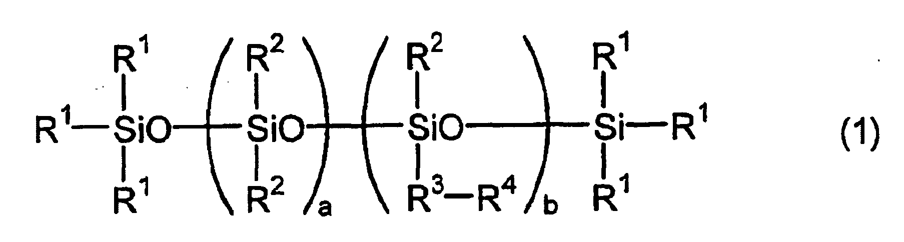 worin R¹ jeweils unabhängig eine monovalente Kohlenwasserstoffgruppe, Hydroxygruppe oder Alkoxygruppe ist, R² jeweils unabhängig eine monovalente Kohlenwasserstoffgruppe ist, R³ jeweils unabhängig eine bivalente C₁₋₁₀-Kohlenwasserstoffgruppe ist, R⁴ jeweils unabhängig eine Gruppe mit der folgenden Formel (2) oder (3) ist 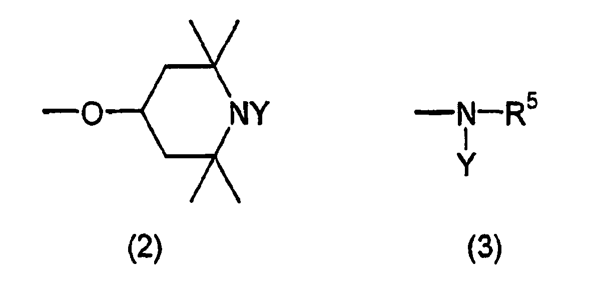 worin Y jeweils unabhängig ein Wasserstoffatom oder eine Gruppe ist: -CH₂CH(OH)-R³-OH (R³ hat die gleiche Bedeutung wie oben beschrieben), R⁵ unabhängig jeweils ein Wasserstoffatom oder eine Gruppe -R³NY₂ ist (Y und R³ haben die gleichen Bedeutungen wie oben beschrieben), worin alle Gruppen Y nicht gleichzeitig ein Wasserstoffatom bedeuten, a für eine Zahl von 25 bis 1000 und b für eine Zahl von 1 bis 200 steht.

7. Haarreinigungszusammensetzung, umfassend die folgenden Komponenten (a), (b) und (c):
(a) von 0,5 bis 60 Gew.-% eines anionischen Tensids,
(b) von 0,1 bis 30 Gew.-% eines Monoalkylglycerylethers oder Monoalkenylglycerylethers mit einer C₄₋₁₂-Alkyl- oder -Alkenylgruppe, umfassend Mischungen davon, und
(c) von 0,05 bis 4 Gew.-% eines Siliconderivates mit einer Gruppe, umfassend sowohl eine Hydroxygruppe als auch ein Stickstoffatom an einer Seitenkette davon, die an ein Siliciumatom gebunden ist.

8. Haarreinigungszusammensetzung nach Anspruch 7, worin das Siliconderivat als Komponente (c) durch die durchschnittliche Formel (1) dargestellt ist 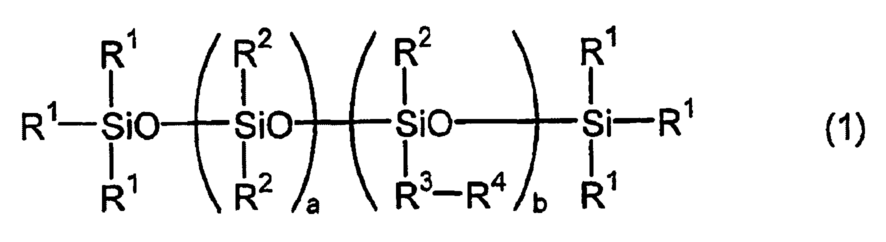 worin R¹ jeweils unabhängig eine monovalente Kohlenwasserstoffgruppe, Hydroxygruppe oder Alkoxygruppe ist, R² jeweils unabhängig eine monovalente Kohlenwasserstoffgruppe ist, R³ jeweils unabhängig eine bivalente C₁₋₁₀-Kohlenwasserstoffgruppe ist, R⁴ jeweils unabhängig eine Gruppe mit der folgenden Formel (2) oder (3) ist 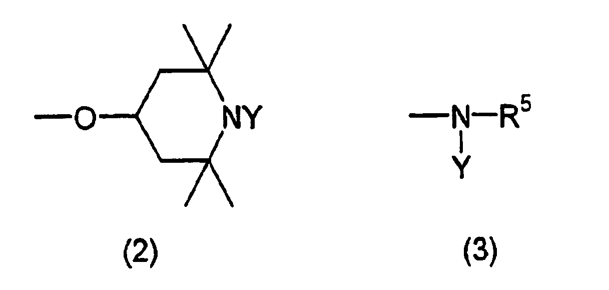 worin Y jeweils unabhängig ein Wasserstoffatom oder eine Gruppe ist: -CH₂CH(OH)-R³-OH (R³ hat die gleiche Bedeutung wie oben beschrieben), R⁵ unabhängig jeweils ein Wasserstoffatom oder eine Gruppe -R³NY₂ ist (Y und R³ haben die gleichen Bedeutungen wie oben beschrieben), worin alle Gruppen Y nicht gleichzeitig ein Wasserstoffatom bedeuten, a für eine Zahl von 25 bis 1000 und b für eine Zahl von 1 bis 200 steht.

## Revendications

1. Composition détergente pour les cheveux, comprenant les constituants (a), (b) et (c) ci-dessous:
(a) un agent tensioactif anionique,
(b) un éther monoalkylique de glycéryle ayant un groupe alkyle en C₄-C₁₂, un éther monoalcénylique de glycéryle ayant un groupe alcényle en C₄-C₁₂, ou leurs mélanges, et
(c) un dérivé de silicone ayant un groupe contenant à la fois un groupe hydroxy et un atome d'azote comme chaîne latérale liée à un atome de silicium.

2. Composition détergente pour les cheveux selon la revendication 1, dans laquelle l'agent tensioactif anionique en tant que constituant (a) est choisi dans le groupe constitué par les types sulfate, sulfonate, carboxylate et leurs mélanges.

3. Composition détergente pour les cheveux selon la revendication 1, dans laquelle l'agent tensioactif anionique en tant que constituant (a) est choisi dans le groupe constitué par RO(CH₂CH₂O)ₙSO₃M, R'OSO₃M et leurs mélanges, où R représente un groupe alkyle ou alcényle en C₁₀-C₁₈, R' représente un groupe alkyle en C₁₀-C₁₈, M représente un métal alcalin, un métal alcalino-terreux, un ammonium, une alcanolamine ou un aminoacide basique, et n représente un nombre de 1 à 5 en moyenne pondérée.

4. Composition détergente pour les cheveux selon la revendication 1, dans laquelle le constituant (b) est un éther monoalkylique de glycéryle ayant un groupe alkyle linéaire en C₄-C₁₀, un éther monoalkylique de glycéryle ayant un groupe alkyle ramifié en C₄-C₁₀, ou leurs mélanges.

5. Composition détergente pour cheveux selon la revendication 4, dans laquelle le groupe alkyle est choisi dans le groupe constitué par les groupes n-butyle, isobutyle, n-pentyle, 2-méthylbutyle, isopentyle, n-hexyle, isohexyle, n-heptyle, n-octyle, 2-éthylhexyle, n-décyle et isodécyle.

6. Composition détergente pour les cheveux selon la revendication 1, dans laquelle le dérivé de silicone, en tant que constituant (c), est représenté par la formule moyenne (1) ci-dessous: 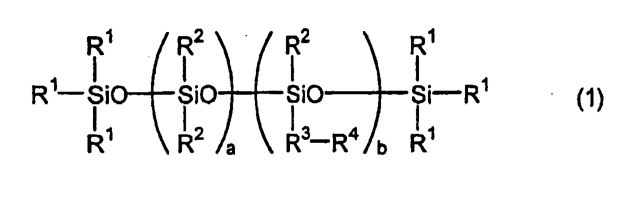 dans laquelle
les R¹ représentent chacun indépendamment un groupe hydrocarboné monovalent, un groupe hydroxy ou un groupe alcoxy,
les R² représentent chacun indépendamment un groupe hydrocarboné monovalent,
les R³ représentent chacun indépendamment un groupe hydrocarboné divalent en C₁-C₁₀,
les R⁴ représentent chacun indépendamment un groupe représenté par la formule (2) ou (3) ci-dessous: 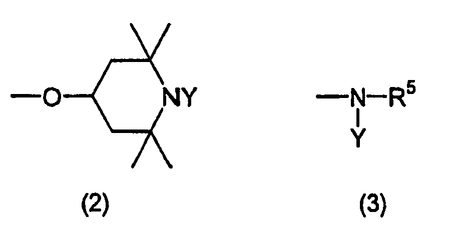 où les Y représentent chacun indépendamment un atome d'hydrogène ou un groupe -CH₂CH(OH)-R³-OH (R³ a la même signification que celle décrite ci-dessus), les R⁵ représentent chacun indépendamment un atome d'hydrogène ou un groupe -R³NY₂ (Y et R³ ont les mêmes significations que celles décrites ci-dessus), tous les Y ne représentant pas en même temps un atome d'hydrogène,
a représente un nombre de 25 à 1 000, et
b représente un nombre de 1 à 200.

7. Composition détergente pour les cheveux comprenant les constituants (a), (b) et (c) ci-dessous:
(a) de 0,5 à 60 % en masse d'un agent tensioactif anionique,
(b) de 0,1 à 30 % en masse d'un éther monoalkylique de glycéryle ou d'un éther monoalcénylique de glycéryle ayant un groupe alkyle ou alcényle en C₄-C₁₂, y compris leurs mélanges, et
(c) de 0,05 à 4 % en masse d'un dérivé de silicone ayant un groupe contenant à la fois un groupe hydroxy et un atome d'azote comme chaîne latérale liée à un atome de silicium.

8. Composition détergente pour les cheveux selon la revendication 7, dans laquelle le dérivé de silicone, en tant que constituant (c), est représenté par la formule moyenne (1) ci-dessous: 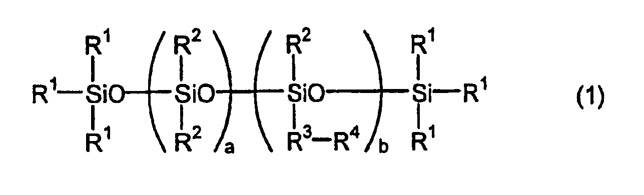 dans laquelle
les R¹ représentent chacun indépendamment un groupe hydrocarboné monovalent, un groupe hydroxy ou un groupe alcoxy,
les R² représentent chacun indépendamment un groupe hydrocarboné monovalent,
les R³ représentent chacun indépendamment un groupe hydrocarboné divalent en C₁-C₁₀,
les R⁴ représentent chacun indépendamment un groupe représenté par la formule (2) ou (3) ci-dessous: 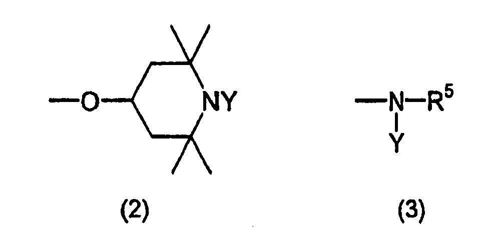 où les Y représentent chacun indépendamment un atome d'hydrogène ou un groupe -CH₂CH(OH)-R³-OH (R³ a la même signification que celle décrite ci-dessus), les R⁵ représentent chacun indépendamment un atome d'hydrogène ou un groupe R³NY₂ (Y et R³ ont les mêmes significations que celles décrites ci-dessus), tous les Y ne représentant pas en même temps un atome d'hydrogène,
a représente un nombre de 25 à 1 000, et
b représente un nombre de 1 à 200.
